# EUROPEAN PATENT APPLICATION

(11) **EP 2 781 580 A1**
(43) Date of publication of application: **24.09.2014**
(21) Application number: 12849331.9
(22) Date of filing: 13.11.2012
(51) Int. Cl.: C10G 3/00, C10L 1/04

(54) **METHOD FOR PRODUCING FUEL OIL**

(30) Priority: 15.11.2011 JP 2011249713
(71) Applicant: Kitakyushu Foundation for the Advancement of Industry, Science and Technology, Kitakyushu-shi, Fukuoka 808-0135 (JP)
(72) Inventor: ASAOKA, Sachio, Kitakyushu-shi Fukuoka 808-0135 (JP); LI, Xiaohong, Kitakyushu-shi Fukuoka 808-0135 (JP); KIMURA, Toshiyuki, Kitakyushu-shi Fukuoka 808-0135 (JP)
(74) Representative: Kling, Simone
(86) International application number: PCT/JP2012/079413
(87) International publication number: WO 2013/073529

(57) **Abstract**

Provided is a method that is for producing fuel oil and that can cheaply and highly efficiently produce a fuel oil-or starting material thereof-having as the primary component n-paraffin or isoparaffin from a starting material oil containing a fatty acid alkyl ester, even while reducing hydrogen pressure. The method for producing fuel oil has a step for producing fuel oil having one or both of n-paraffin and isoparaffin as the primary component by contacting hydrogen gas and a starting material oil containing a fatty acid alkyl ester under the condition of a hydrogen pressure of no greater than 1 MPa to a catalyst resulting from supporting on a porous metal oxide support one or more metal elements belonging to group nine or group ten of the periodic table, and one or more group six element oxides belonging to group six of the periodic table. The weight ratio of the group six elements to the metal elements contained in the catalyst is no greater than 1.0 in terms of the metal.

## Description

### TECHNICAL FIELD

The present invention relates to a method for producing a fuel oil from fatty acid alkyl ester used in biodiesel fuel (BDF), and more particularly, to a method for producing fuel oil that is useful as an aviation fuel.

### BACKGROUND ART

Biomass fuels, which are non-exhaustible resources that do not cause an increase in the concentration of carbon dioxide in the atmosphere (i.e., are carbon neutral), are attracting attention as fuel oil raw materials to take the place of conventional petroleum from the viewpoints of growing social demand for reducing levels of greenhouse gases, escalating crude oil prices and the need to conserve petroleum resources.

Known examples of biofuels produced from biomass raw materials include bioalcohol fuels obtained by direct fermentation of sugars contained in sugar cane or corn or by fermentation of sugars obtained by hydrolyzing cellulose contained in sustainable wood, and biodiesel fuels (BDF) that use fatty acid methyl esters obtained by transesterification of animal and vegetable oils as fuel oil. Among these, bioalcohol fuels using sugar cane or corn as raw materials are associated with problems such as having an effect on the stable supply of foodstuffs, requiring considerable energy for removal of water, and being difficult to apply to aviation fuel. Bioalcohol fuels using cellulose as raw materials are associated with problems such as high production costs and also being difficult to apply to aviation fuel.

Since biodiesel fuel is used by adding to or mixing with conventional petroleum-based fuels (see, for example, Patent Document 1), in addition to still being inadequate as a completely alternative technology to petroleum-based raw materials, it is also associated with problems such as deterioration caused by oxygen and freezing at low temperatures. In addition, since it is necessary to process the glycerin produced as a byproduct as well as clean the oil formed, high production costs are currently a barrier to its proliferation in the transport industry amidst increasingly intense price competition.

Moreover, since biodiesel fuel also contains a large number of carbon atoms in the fatty acid groups that compose the oil while also consisting of linear molecules, it also has the shortcoming of preventing the obtaining of a sufficiently high octane rating.

In consideration of the aforementioned problems, biohydrocracking fuels (BHF) have been proposed that consist mainly of hydrocarbon-based compounds and are obtained by a process consisting of decomposing plant and vegetable oils in the presence of hydrogen gas and catalyst using a vacuum-distilled gas oil hydrocracking device and the like. During the cracking process, reactions occur that include reduction of carboxyl groups, shortening of hydrocarbon chains and isomerization of linear alkyl groups to branched alkyl groups, thereby resulting in the obtaining of a mixture composed of hydrocarbon compounds having a desired number of carbon atoms and branching (see Patent Documents 2 to 7).

### Prior Art Documents

### Patent Documents

Patent Document 1: Japanese Unexamined Patent Application Publication (Translation of PCT Application) No. 2010-532419
Patent Document 2: Japanese Unexamined Patent Publication No. 2009-40833
Patent Document 3: Japanese Unexamined Patent Publication No. 2009-40855
Patent Document 4: Japanese Unexamined Patent Publication No. 2009-40856
Patent Document 5: Japanese Unexamined Patent Application Publication (Translation of PCT Application) No. 2011-515539
Patent Document 6: Japanese Unexamined Patent Publication No. 2011-52074
Patent Document 7: Japanese Unexamined Patent Publication No. 2011-52077

### DISCLOSURE OF THE INVENTION

### Problems to be Solved by the Invention

However, since the production of gas oil compositions described in Patent Documents 2 to 4, 6 and 7 is carried out at a high hydrogen pressure of 2 MPa to 13 MPa thereby requiring a pressure vessel, these gas oil compositions have the problem of excessively high production costs.

Production of transportation fuel described in Patent Document 5 also requires a high hydrogen pressure of about 0.7 MPa to about 14 MPa during hydrogenation treatment.

With the foregoing in view, an object of the present invention is to provide a method for producing base oil enabling the inexpensive and high-yield production of fuel oil, or raw material thereof, composed mainly of n-paraffin or isoparaffin, from base oil containing fatty acid triglyceride.

### Means for Solving the Problems

The present invention solves the aforementioned problems by providing a method for producing fuel oil described in any of [1] to [12] below.
[1] A method for producing fuel oil, comprising: a step for producing a fuel oil composed mainly of one or both of n-paraffin and isoparaffin by contacting a base oil containing fatty acid alkyl ester and hydrogen gas with a catalyst, obtained by supporting one or a plurality of metal elements belonging to group 9 or group 10 of the periodic table and one or a plurality of group 6 element oxides belonging to group 6 of the periodic table on a porous metal oxide support, under conditions of a hydrogen pressure of 1 MPa or less; wherein, the weight ratio as metal of the group 6 element contained in the catalyst to the metal element does not exceed 1.0.
[2] The method for producing fuel oil described in [1] above, wherein the metal element is nickel and/or cobalt, and the group 6 element is molybdenum and/or tungsten-molybdenum.
[3] The method for producing fuel oil described in [2] above, wherein the metal element is nickel and the group 6 element is molybdenum.
[4] The method for producing fuel oil described in any of [1] to [3], wherein the porous metal oxide support is γ-alumina or a modification product thereof.
[5] The method for producing fuel oil described in any of [1] to [4] above, wherein the base oil, the hydrogen gas and the catalyst are contacted under conditions of a liquid hourly space velocity of 0.5 hr⁻¹ to 20 hr⁻¹ and a reaction temperature of 250°C to 400°C.
[6] The method for producing fuel oil described in any of [1] to [5] above, wherein the content of saturated fatty acid groups having 8 to 14 carbon atoms in the fatty acid group composition of fatty acid alkyl ester contained in the base oil is 40% by weight or more.
[7] The method for producing fuel oil described in [6] above, wherein the content of lauric acid groups in the fatty acid group composition of the fatty acid alkyl ester is 40% by weight or more.
[8] The method for producing fuel oil described in any of [1] to [7] above, wherein the base oil is produced from an oil derived from a plant or bacteria.
[9] The method for producing fuel oil described in [8] above, wherein the oil derived from a plant is a mixture of oils derived from two or more types of plants.
[10] The method for producing fuel oil described in [8] or [9] above, wherein the oil derived from a plant is coconut oil, palm kernel oil or a mixture thereof.
[11] The method for producing fuel oil described in [8] or [9] above, wherein the oil derived from a plant is oil derived from algae.
[12] The method for producing fuel oil described in any of [1] to [11], wherein the resulting fuel oil satisfies the requirements for aviation fuel defined in ASTM D 7566.

### Effects of the Invention

According to the present invention, a fuel oil composed mainly of fuel oil raw materials in the form of one or both of n-paraffin and isoparaffin can be produced inexpensively and at high yield by hydrocracking fatty acid alkyl ester contained in a base oil at a low hydrogen pressure of 1 MPa or less. Consequently, high-quality fuel oil can be produced at low cost from carbon-neutral raw materials such as vegetable oil. Thus, a fuel oil can be provided as a useful alternative to conventional fossil fuel-derived fuel oils, thereby making it also possible to present effective solutions for depletion of fossil fuels as well as environmental issues such as the reduction of greenhouse gases as exemplified by carbon dioxide.

### BRIEF DESCRIPTION OF THE DRAWINGS

FIG. 1 is a graph indicating the relationship between reaction time and conversion rate during hydrocracking of fatty acid methyl ester derived from coconut oil.
FIG. 2 is a graph indicating the distribution of the carbon number of hydrocarbons obtained by hydrocracking of fatty acid methyl ester derived from coconut oil.

### BEST MODE FOR CARRYING OUT THE INVENTION

Next, an explanation is provided of specific embodiments for embodying the present invention.

The catalyst used to carry out the method for producing fuel oil of the present invention (to be simply referred to as the "catalyst") is obtained by supporting one or a plurality of types of metal elements belonging to group 9 or group 10 of the periodic table and a one or a plurality of types of group 6 element oxides belonging to group 6 of the periodic table on a porous metal oxide support.

A support containing alumina or a compound oxide of silica and the like mainly composed of alumina can be used for the porous metal oxide support. Among these, alumina or modified alumina mainly composed of alumina is preferable from the viewpoint of increasing the specific surface area of the catalyst. Although porous γ-alumina (γ-Al₂O₃) is particularly preferable for the aforementioned alumina, α-alumina, β-alumina or amorphous alumina may also be used.

The alumina serving as the main component of the support may be produced using any of a method consisting of heat-treating aluminum hydroxide obtained by neutralizing an aluminum salt with base, a method consisting of neutralizing or hydrocracking an aluminum salt and aluminate, or a method consisting of hydrolyzing an aluminum amalgam or aluminum alcoholate, and may also be produced using a commercially available alumina intermediate or boehmite powder and the like as a precursor in addition to the methods described above.

In addition to alumina, the porous inorganic oxide support may also contain silica (SiO₂), silica-alumina (SiO₂/Al₂O₃), boria (B₂O₃), titania (TiO₂), magnesia (MgO), activated carbon, diphosphorus pentoxide (P₂O₅) or a compound oxide thereof.

Moreover, the aforementioned porous inorganic oxide support may also contain zeolite as silica-alumina (SiO₂/Al₂O₃). Zeolite is the generic term for aluminosilicate having fine pores in the crystals thereof, and specific examples thereof include natural zeolite compounds such as amicite (monoclinic system), ammonioleucite, analcime, berbergate, bikitaite, bauxite, chabazite, sodium chabazite, potassium chabazite, oblique protyl chabazite, oblique protyl potassium chabazite, oblique protyl sodium chabazite, oblique protyl calcium chabazite, cowlesite, calcium dachiardite, sodium dachiardite, edingtonite, epistilbite, erionite, sodium erionite, potassium erionite, calcium erionite, ferrierite, magnesium ferrierite, potassium ferrierite, sodium ferrierite, garronite, gismondite, gmelinite, sodium gmelinite, calcium gmelinite, potassium gmelinite, gobbinsite, gonnardite, harmotome, heulandite, calcium heulandite, strontium-lithium heulandite, sodium heulandite, potassium heulandite, laumontite, leucite, calcium levyne, sodium levyne, mesolite, sodium zeolite, phillipsite, sodium phillipsite, potassium phillipsite, calcium phillipsite, pollucite, scolecite, stellerite, stilbite, calcium stilbite, sodium stilbite, thomsonite, wairakite or yugawaralite, and synthetic zeolite compounds such as type A, zeolite, type Y zeolite, type X zeolite, type beta zeolite or ZSM-5 zeolite.

In addition, in the case of using zeolite for the porous metal oxide support, there are no particular limitations on the structure thereof, and may be type Y zeolite, type X zeolite, type beta zeolite or ZSM-5 zeolite and the like, or may be a mixture containing two or more arbitrary types thereof.

"Group 9 or group 10 of the periodic table" respectively refers to group 9 or group 10 of the long periodic table (IUPAC periodic table), and specific examples of metal elements belonging to these groups include cobalt (Co), nickel (Ni), rhodium (Rh), palladium (Pd), iridium (Ir) and platinum (Pt). Among these metal elements, cobalt and nickel are preferable in terms of catalytic activity and price, and nickel is particularly preferable. The catalyst contains one type of two or more arbitrary types of these metals, and is supported on the surface of the porous metal oxide support in the form of a metal.

"Group 6 of the periodic table" refers to group 6 of the long periodic table (IUPAC periodic table), and specific examples of elements belonging to this group (referred to as "group 6 elements" in the present invention) include chromium (Cr), molybdenum (Mo) and tungsten (W). Among these, molybdenum and tungsten are preferable, and molybdenum is particularly preferable. The catalyst contains one type or two or more arbitrary types of these metals, and is supported on the surface of the porous metal oxide support in the form of an oxide.

Metal elements belonging to group 9 or group 10 of the periodic table have catalytic activity in a hydrocracking reaction of fatty acid alkyl ester contained in the base oil, and oxides of elements belonging to group 6 of the periodic table are thought to impart basicity to the catalyst and contribute to improvement of dispersibility of the aforementioned metals. The weight ratio as metal of a group 6 element to a metal element belonging to group 9 or group 10 of the periodic table does not exceed 1.0. Namely, in the case of defining the weight of a metal element belonging to group 9 or group 10 of the periodic table as w₁ and defining the weight of a group 6 element as metal as w₂, then (w₂/w₁) ≤ 1. If w₂/w₁ exceeds 1, conversion efficiency and hydrocarbon yield of the base oil decrease due to a decrease in hydrocracking activity. The preferable range of w₂/w₁ is 0.05 to 1.0, more preferably 0.1 to 0.7, and even more preferably 0.15 to 0.5.

The catalyst is produced by carrying out hydrogen reduction treatment after impregnating an aqueous solution containing a salt of a group 6 element and an aqueous solution containing a salt of a metal belonging to group 9 or group 10 of the periodic table into a porous metal oxide support and firing. There are no particular limitations on the salts used to produce the catalyst provided they are soluble in water, and salts of inorganic acids such as nitrates, halides, sulfates or phosphates, or salts of organic acids such as carboxylates, can be used. Furthermore, a salt of a polyacid or a salt of a heteropolyacid are used preferably for the salt of the group 6 element since they can be acquired comparatively inexpensively. Although a salt of a group 6 element and a salt of a metal belonging to group 9 or group 10 of the periodic table may be supported simultaneously by impregnating an aqueous solution containing both followed by firing and carrying out hydrogen reduction treatment, they may also be supported by impregnating one followed by firing and hydrogen reduction and then impregnating the other followed by firing and hydrogen reduction treatment. Alternatively, hydrogen reduction treatment may be carried out after having carried out impregnation and firing in that order.

A fatty acid alkyl ester synthesized from an arbitrary animal oil or vegetable oil containing fatty acid triglyceride as the main component thereof and a lower alkyl alcohol using an arbitrary known method such as transesterification in the presence of an acid or base catalyst can be used without any particular limitations for base oil used in the production of fuel oil.

Although examples of lower alkyl alcohols used to produce the fatty acid alkyl ester include methanol, ethanol, n-propyl alcohol, isopropyl alcohol, n-butanol, isobutyl alcohol and t-butyl alcohol, methanol is used most preferably. In addition, examples of catalysts used in the transesterification reaction include acid catalysts such as hydrochloric acid, sulfuric acid, alkylsulfonic acid or arylsulfonic acid, solid acid catalysts such as Nafion-H (trade name), and base catalysts such as sodium hydroxide, potassium hydroxide, sodium alkoxide, potassium alkoxide or lanthanoid alkoxides.

Since the fatty acid group composition (carbon number and degree of unsaturation of fatty acid groups) of fatty acid alkyl ester contained in the base oil has an effect on the carbon number and content of each hydrocarbon compound that composes the resulting fuel oil, a suitable base oil can be suitably selected and used corresponding to the required performance, carbon number and so forth of the desired base oil. Specific examples of oils and fats used to obtain base oil include vegetable oils such as corn oil, soybean oil, sesame oil, rapeseed oil (canola oil), rice oil, rice bran oil, camellia oil, safflower oil (safflower seed oil), palm kernel oil, coconut oil, cottonseed oil, sunflower oil, perilla oil, olive oil, peanut oil, almond oil, avocado oil, hazelnut oil, walnut oil, grape seed oil, mustard oil, lettuce oil, cocoa butter, palm oil or oils produced by algae such as marine algae or microalgae, animal oils and fats such as fish oil, whale oil, shark oil, liver oil, lard (pork tallow), beef drippings (beef tallow), chicken oil, rabbit tallow, mutton tallow, horse fat, milk fat or butter, and fats and oils produced by bacteria.

Many of the aforementioned animal and vegetable oils are derived from food raw materials in the form of agricultural crops and livestock, and there are some that present problems from the viewpoint of ensuring a stable supply due to problems involving competition over food resources and difficulty in large-scale cultivation. Therefore, oils such as jatropha oil and other non-edible oils as well as oils derived from microalgae or bacteria can be used as base oils that do not compete with food, and in particular, oil may be used that is derived from microalgae, which have superior propagative power, demonstrate a high production output of oil per unit volume, and can be easily cultivated on a large scale. The use of base oil derived from microalgae offers the advantages of being able to significantly reduce base oil procurement costs and transport costs, while also making it possible to hold down the price of fuel oil to a low level.

Examples of microalgae able to be used as supply sources of base oil include Botoryococcus braunii, Chlorella species and Aurantiochytrium species. An example of a base oil derived from microalgae composed mainly of fatty acid alkyl ester suitable for the production of aviation fuel having a carbon number of 8 to 14 and saturated fatty acid group content of 40% by weight or more in the fatty acid group composition thereof is fatty acid alkyl ester produced from oil derived from microalgae deposited under accession number FERM P-22090.

Among fuel oils, aviation fuel is required to consist mainly of n-paraffin and isoparaffin having a carbon number of about 8 to 14 and demonstrate superior low-temperature properties (with respect to cloud point, solidification temperature and the like). Base oil for efficiently producing fuel oil able to satisfy these requirements is preferably a base oil composed mainly of fatty acid alkyl ester in which the content of saturated fatty acid groups having 8 to 14 carbon atoms in the fatty acid group composition of fatty acid alkyl ester contained in the base oil is 40% by weight or more, and particularly preferably that in which the content of lauric acid groups is 40% by weight or more. Specific examples of the aforementioned animal and vegetable oils that are preferable as raw materials of aviation fuel include fatty acid alkyl esters derived from coconut oil harvested from coconut seeds and palm kernel oil harvested from African oil palm trees, as well as fatty acid alkyl esters derived from oil harvested from algae, and particularly microalgae.

In the case of a high free fatty acid content of the base oil, although pretreatment for removing free fatty acids may be carried out as necessary, since free fatty acids also form fatty acid alkyl esters by reacting with lower alkyl alcohols under the same conditions as esterification of fatty acid triglycerides, base oils containing free fatty acids can normally be used as base oils for the production of fuel oil without having to separate and remove the free fatty acids.

Fuel oil can be efficiently obtained at a low hydrogen pressure of 1 MPa or lower and preferably 0.8 MPa or lower, and at a comparatively low reaction temperature of 250°C to 400°C, by using the aforementioned catalyst and base oil containing fatty acid alkyl ester in combination. Since hydrogen pressure can be lowered, it is not necessary to use a reaction vessel having high pressure resistance, and since there is also relatively little susceptibility to the effects of hydrogen embrittlement in reaction vessels made of metal, there are fewer restrictions on production equipment and fuel oil can be produced at low cost.

The liquid hourly space velocity (LHSV) during the reaction is, for example 0.5 hr⁻¹ to 20 hr⁻¹, and the hydrogen/oil ratio is, for example, 50 NL/L to 4000 NL/L. These values are suitably adjusted corresponding to such factors as the composition of the base oil and the required performance of the fuel oil (e.g., carbon number, low-temperature flow properties).

Fuel oil obtained in this manner has for a main component thereof n-paraffin having a carbon number roughly equal to the carbon number of the fatty acid groups contained in the base oil. In cases in which it is necessary to improve the content of isoparaffin, isomerization treatment may be carried out using any known catalyst such as a platinum catalyst or solid acid catalyst. Furthermore, in the case of using γ-alumina and the like for the porous metal oxide support, there are cases in which the formation of isoparaffin is observed due to isomerization proceeding simultaneously as a result of this acting as a solid acid catalyst. In such cases, the amount of time required for the isomerization step required for improving isoparaffin content can be shortened, and depending on the case, the isomerization step can be omitted. Consequently, fuel oil production costs can be reduced particularly in cases in which it is necessary to improve isoparaffin content.

Fuel oil can also be obtained by suitably adding additives such as antioxidants or anti-freezing agents as necessary. In the case of using coconut oil or oil derived from microalgae as base oil in particular, fuel oil is obtained that satisfies the requirements for aviation fuel defined in ASTM D 7566.

The main regulations relating to aviation turbine fuel oil containing synthetic hydrocarbons as defined in ASTM D 7566 (American Society for Testing and Materials) are as indicated below.
* Hydrocarbon oils: 99.5% or more
* Cycloparaffins: 15% or less
* Paraffin-based hydrocarbon oils: 70% to 85%
* Olefin-based hydrocarbons: 5% or less
* Aromatic compounds: 0.5% or less
* Acidity: 0.10 mgKOH/g or less
* Sulfur compounds: 3 ppm or less

### Examples

The following provides an explanation of examples carried out to confirm the action and effects of the present invention.

### Example 1: Catalyst Preparation

### <1> Preparation of γ-Al₂O₃

3900 cc of an aqueous aluminum nitrate solution having a concentration of 2.67 mol/L and 3900 cc of an aqueous ammonia solution having a concentration of 14% by weight were prepared. Next, a pH swing procedure was repeated six times consisting of placing 20 L of pure water in a 30 L porcelain enameled container followed by heating to 70°C while stirring, continuing to stir while injecting 650 cc of the aforementioned aqueous aluminum nitrate solution and stirring for 5 minutes (pH value: 2.0), and injecting 650 cc of the aforementioned aqueous ammonia solution and stirring for 5 minutes (pH 7.4). A washing procedure, consisting of recovering a cake by filtering the resulting aqueous aluminum hydroxide slurry followed by re-dispersing the cake in 20 L of pure water and filtering again, was repeated three times to obtain a washed cake of the aluminum hydroxide. Next, after adjusting the water content of the washed cake by drying, the cake was molded into the shape of rods having a diameter of 1.6 mm with an extrusion molding machine, and after drying under conditions of 120°C for 3 hours, the molded rods were crushed to a length of about 1 cm followed by firing in a muffle furnace under conditions of 500°C for 3 hours to obtain a γ-alumina support.

The specific surface area of the resulting γ-alumina support was 275 m²/g, the pore volume was 0.65 cc/g, the mean pore diameter was 8.9 nm (89 Å), and the ratio of pores having a pore diameter within ±3 nm (30 Å) of the mean pore diameter to the total pore volume was 91%. The pore size distribution of the γ-alumina support obtained according to this method was extremely small, and the support was able to be confirmed to have a porous structure consisting of pores having a uniform pore diameter.

### <2> Preparation of Hydrocracking Catalyst (1)

After impregnating 100 g of the aforementioned porous inorganic oxide support into 97 cc of an aqueous nickel nitrate solution adjusted to a concentration of 0.5 mol/L using the aforementioned porous inorganic oxide support and allowing to stand undisturbed for 12 hours in a sealed container, moisture was removed at normal temperature with an evaporator followed by firing in air with an electric furnace under conditions of 200°C for 3 hours to obtain various fired products in which nickel (Ni) was supported on a porous inorganic oxide support. Next, each of the resulting fired products was filled into a flow-type hydrogen reduction device followed by carrying out hydrogen reduction in the presence of a hydrogen flow under conditions of 370°C for 15 hours to obtain a hydrocracking catalyst (1).

### <3> Preparation of Hydrocracking Catalyst (2)

After impregnating an aqueous solution, obtained by dissolving 7. 19 g of ammonium molybdate [(NH₄) ₆MO₇O₂₄·4H₂O] in 65.92 cc of pure water, into 100 g of the hydrocracking catalyst (1) obtained in the manner described above and allowing to stand undisturbed for 12 hours in a sealed container, moisture was removed at normal temperature with an evaporator followed by firing with an electric furnace in air under conditions of 200°C for 3 hours, filling the fired product into a flow-type hydrogen reduction device, and carrying out hydrogen reduction in the presence of flowing hydrogen under conditions of 370°C for 15 hours to prepare a hydrocracking catalyst (2) in which molybdic acid (MO₃) was added at a ratio of 5% based on the aforementioned hydrocracking catalyst.

The supported amount of nickel as nickel metal (Ni supported amount) in the resulting hydrocracking catalyst (2) was 22% by weight, and the supported amount of molybdic acid (MoO₃) (MoO₃ supported amount) was 5% by weight.

### <4> Preparation of Hydrocracking Catalyst (3)

After impregnating an aqueous solution, obtained by dissolving 4.72 g of ammonium tungstenate [5(NH₄)₂·12WO₃·5H₂O] in 65.92 cc of aqueous aminoethanol solution, into 100 g of the hydrocracking catalyst (1) obtained in the manner described above and allowing to stand undisturbed for 12 hours in a sealed container, moisture was removed at normal temperature with an evaporator followed by firing with an electric furnace in air under conditions of 200°C for 3 hours, filling the fired product into a flow-type hydrogen reduction device, and carrying out hydrogen reduction in the presence of flowing hydrogen under conditions of 370°C for 15 hours to prepare a hydrocracking catalyst (3) in which tungstic acid (WO₃) was added at a ratio of 5% based on the hydrocracking catalyst (1). The supported amount of nickel as nickel metal (Ni supported amount) in the resulting hydrocracking catalyst (3) was 15% by weight, and the supported amount of tungstic acid (WO₃) (WO₃ supported amount) was 5% by weight.

### Example 2: Production of Fuel Oil Using Coconut Oil-Derived Fatty Acid Methyl Ester as Base Oil

### <1> Production of Fatty Acid Methyl Ester from Coconut Oil

Hybrid coconut oil was reacted with methanol in the presence of a catalyst (arbitrary known acid catalyst such as sulfuric acid or p-toluenesulfonic acid, or arbitrary known base catalyst such as sodium hydroxide or potassium hydroxide) to synthesize a fatty acid methyl ester by a transesterification reaction. The resulting fatty acid methyl ester contained as main components thereof 45% by weight to 52% by weight of methyl laurate (12:0), 15% by weight to 22% by weight of methyl myristate (14:0), 6% by weight to 10% by weight of methyl caprylate (8:0), 4% by weight to 12% by weight of methyl caprate (10:0), 1% by weight to 5% by weight of methyl stearate (18:0), 2% by weight to 10% by weight of methyl oleate (18:1) and 1% by weight to 3% by weight of methyl linoleate (18:2). (Furthermore, numbers shown in parentheses indicate the carbon number and number of double bonds.)

### <2> Production of Fuel Oil by Hydrocracking of Coconut Oil-Derived Fatty Acid Methyl Ester

Hydrocracking was carried out under the following conditions using coconut oil-derived fatty acid methyl ester synthesized in the manner describe above as base oil.
* Reaction temperature: 350°C
* LHSV: 1.0 h⁻¹
* Reaction pressure: 0.8 MPa
* H₂/fatty acid methyl ester flow rate ratio: 1250 NL/L
* Amount of catalyst: 2.0 mL
* Catalyst particle diameter: 355 µm to 600 µm

Furthermore, the catalyst used was the hydrocracking catalyst (2) prepared in section <3> of the aforementioned Example 1, and pretreatment in the form of hydrogen reduction treatment was carried out for 7 hours at 370°C and GHSV = 5000 h⁻¹.

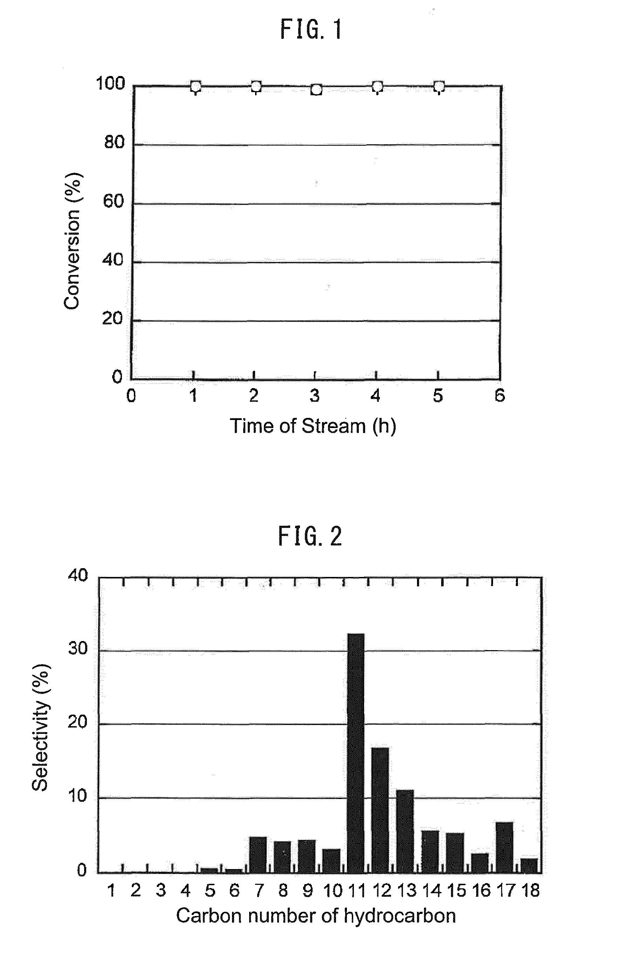

The relationship between reaction time and conversion rate is shown in FIG. 1, while the distribution of the carbon numbers of the resulting hydrocarbons is shown in FIG. 2. As is shown in FIG. 1, the conversion rate in this reaction demonstrated a value of nearly 100%, while as shown in FIG. 2, the products consisted mainly of C₁₁ hydrocarbons, and nearly all of those C₁₁ hydrocarbons consisted of normal paraffin. In addition, although the results of calculating the yields of the resulting liquid hydrocarbons, the yield of the aviation fuel fraction indicating the ratio of aviation fuel fractions (C₇ to C₁₆) among liquid hydrocarbons, and the average carbon number of the liquid hydrocarbons are shown in the following Table 1, both liquid hydrocarbon yield and aviation fuel fraction yield were extremely high at 94.5% and 90.5%, respectively, thereby confirming that results were able to be obtained that are comparable to the case of using fatty acid methyl ester obtained from microalgae-derived oil as base oil. In addition, cloud point, acidity and sulfur compound content all satisfied the regulations of ASTM D 7566.

**Table 1: Conversion Rate, Paraffin Content, Olefin Content, Liquid Hydrocarbon Yield, Aviation Fuel Fraction Yield and Average Carbon Number**

| | |
|---|---|
| Conversion rate (%) | 100.0 |
| Paraffin content (wt%) | 100.0 |
| Olefin content (wt%) | 0.0 |
| Liquid hydrocarbon yield (%) | 94.5 |
| Aviation fuel fraction yield (%) | 90.5 |
| Aviation fuel fraction average carbon number | 10.1 |

### Example 3: Production of Fuel Oil Using Microalgae-Derived Fatty Acid Methyl Ester as Base Oil

### <1> Production of Fatty Acid Methyl Ester from Microalgae-Derived Oil

Microalgae deposited under accession number FERM P-22090 were cultured, and the harvested oil (to be referred to as "microalgae oil") was reacted with methanol in the presence of a catalyst (arbitrary known acid catalyst such as sulfuric acid or p-toluenesulfonic acid, or arbitrary known base catalyst such as sodium hydroxide or potassium hydroxide) to synthesize a fatty acid methyl ester by a transesterification reaction. The fatty acid group composition of the resulting fatty acid ester (as analyzed by GC/MS analysis) was as shown in the following Table 2, and the ratio of lauric acid (C₁₁H₂₃COOH) in the fatty acid group composition was determined to be 40% by weight or more.

**Table 2: Fatty Acid Group Composition of Microalgae Oil-Derived Fatty Acid Methyl Ester**

| Fatty acid groups in microalgae oil-derived fatty acid methyl ester | (wt%) |
|---|---|
| Caprylic acid groups (8:0) | 4.6 |
| Capric acid groups (10:0) | 5.0 |
| Lauric acid groups (12:0) | 48.1 |
| Myristic acid groups (14:0) | 19.7 |
| Palmitic acid groups (16:0) | 10.3 |
| Stearic acid groups (18:0) | 4.1 |
| Oleic acid groups (18:1) | 8.1 |
| Linoleic acid groups (18:2) | 0.0 |
| Linolenic acid groups (18:3) | 0.0 |

### <2> Production of Fuel Oil by Hydrocracking of Microalgae Oil-Derived Fatty Acid Methyl Ester

Hydrocracking was carried out in the same manner as section <2> of the aforementioned Example 2 using microalgae oil-derived fatty acid methyl ester as base oil. The conversion rate, paraffin and isoparaffin contents, liquid hydrocarbon yield, aviation fuel fraction yield and aviation fuel fraction average carbon number are shown in Table 3. In addition, the results of measuring the carbon number distribution in the fuel oil are shown in Table 4.

**Table 3: Reaction Results Using Microalgae Oil-Derived Fatty Acid Methyl Ester as Base Oil**

| Conversion rate (%) | 100.0 |
|---|---|
| Paraffin content (wt%) | 100.0 |
| Olefin content (wt%) | 0.0 |
| Liquid hydrocarbon yield (%) | 94.6 |
| Aviation fuel fraction yield (%) | 91.5 |
| Aviation fuel yield average carbon number | 10.0 |

**Table 4: Product Carbon Number Ratios**

| Carbon number | Raw material (wt%) | Product (wt%) |
|---|---|---|
| C₄ | 0 | 0 |
| C₅ | 0 | 0.6 |
| C₆ | 0 | 0.5 |
| C₇ | 0 | 4.8 |
| C₈ | 4.6 | 4.2 |
| C₉ | 0 | 4.4 |
| C₁₀ | 5.0 | 3.2 |
| C₁₁ | 0 | 32.3 |
| C₁₂ | 48.1 | 16.8 |
| C₁₂ | 0 | 11.1 |
| C₁₄ | 19.7 | 5.6 |
| C₁₅ | 0 | 5.3 |
| C₁₆ | 10.3 | 2.6 |
| C₁₇ | 0 | 6.7 |
| C₁₈ | 12.2 | 1.9 |

As shown in Table 3, in the case of using hydrocracking catalyst (2) prepared in section <3> of Example 1, extremely high hydrocarbon yield and aviation fuel fraction yield were able to be obtained. In addition, liquid hydrocarbon yield, aviation fuel fraction (C₇ to C₁₆) selectivity among liquid hydrocarbons and the average carbon number were calculated based on a value of 100% for the yield assuming deoxygenation of all raw materials. As shown in Table 4, products were distributed centering on C₁₁, odd-numbered and even-numbered hydrocarbons were formed, and the ratio of odd-numbered hydrocarbons to even-numbered hydrocarbons was confirmed to be roughly 2:1. Although nearly all of the products consisted of normal paraffin, since normal paraffin is a liquid at normal temperatures, there were not thought to be any problems with respect to fluidity. In addition, precipitation of wax crystals was not observed even when cooled to -40°C. Values satisfying the regulations of ASTM D 7566 were measured for acidity and sulfur content.

Furthermore, although fatty acid methyl ester obtained by transesterification of fatty acid triglyceride harvested by culturing microalgae deposited under accession number FERM P-22090 using methanol in the presence of catalyst was used as base oil in Example 3, a fatty acid methyl ester mixture having a composition as shown in Table 2, for example, may also be prepared and used as a base oil by blending fatty acid methyl esters obtained from oils derived from two or more known types of plants (which may be algae, including microalgae, or bacteria) in an arbitrary ratio.

## Claims

1. A method for producing fuel oil, comprising:
a step for producing a fuel oil composed mainly of one or both of n-paraffin and isoparaffin by contacting:
a base oil containing fatty acid alkyl ester, and
hydrogen gas
with a catalyst, obtained by supporting one or a plurality of metal elements belonging to group 9 or group 10 of the periodic table and one or a plurality of group 6 element oxides belonging to group 6 of the periodic table on a porous metal oxide support, under conditions of a hydrogen pressure of 1 MPa or less; wherein,
the weight ratio as metal of the group 6 element contained in the catalyst to the metal element does not exceed 1.0.

2. The method for producing fuel oil according to claim 1, wherein the metal element is nickel and/or cobalt, and the group 6 element is molybdenum and/or tungsten.

3. The method for producing fuel oil according to claim 2, wherein the metal element is nickel and the group 6 element is molybdenum.

4. The method for producing fuel oil according to any of claims 1 to 3, wherein the porous metal oxide support is γ-alumina or a modification product thereof.

5. The method for producing fuel oil according to any of claims 1 to 4, wherein the base oil, the hydrogen gas and the catalyst
are contacted under conditions of a liquid hourly space velocity of 0.5 hr⁻¹ to 20 hr⁻¹ and a reaction temperature of 250°C to 400°C.

6. The method for producing fuel oil according to any of claims 1 to 5, wherein the content of saturated fatty acid groups having 8 to 14 carbon atoms in the fatty acid group composition of fatty acid alkyl ester contained in the base oil is 40% by weight or more.

7. The method for producing fuel oil according to claim 6, wherein the content of lauric acid groups in the fatty acid group composition of the fatty acid alkyl ester is 40% by weight or more.

8. The method for producing fuel oil according to any of claims 1 to 7, wherein the base oil is produced from an oil derived from a plant or bacteria.

9. The method for producing fuel oil according to claim 8, wherein the oil derived from a plant is a mixture of oils derived from two or more types of plants.

10. The method for producing fuel oil according to claim 8 or claim 9, wherein the oil derived from a plant is coconut oil, palm kernel oil or a mixture thereof.

11. The method for producing fuel oil according to claim 8 or claim 9, wherein the oil derived from a plant is oil derived from algae.

12. The method for producing fuel oil according to any of claims 1 to 11, wherein the resulting fuel oil satisfies the requirements for aviation fuel defined in ASTM D 7566.
